(19) Europäisches Patentamt
European Patent Office
Office européen des brevets

(11) Veröffentlichungsnummer: **0 278 040 B1**

(12) **EUROPÄISCHE PATENTSCHRIFT**

(45) Veröffentlichungstag der Patentschrift: **14.08.91**

(51) Int. Cl.⁵: **A61K 31/505**, A61K 31/52

(21) Anmeldenummer: **87102094.7**

(22) Anmeldetag: **13.02.87**

Die Akte enthält technische Angaben, die nach dem Eingang der Anmeldung eingereicht wurden und die nicht in dieser Patentschrift enthalten sind.

# (54) Verwendung von Pteridinen und/oder Purinen oder eines Xanthinoxidase-Hemmers zur Herstellung eines Arzneimittels zur Behandlung von genetisch bedingten degenerativen Netzhauterkrankungen.

(43) Veröffentlichungstag der Anmeldung:
**17.08.88 Patentblatt 88/33**

(45) Bekanntmachung des Hinweises auf die Patenterteilung:
**14.08.91 Patentblatt 91/33**

(84) Benannte Vertragsstaaten:
**AT BE CH DE ES FR GB IT LI NL SE**

(56) Entgegenhaltungen:
**US-A- 4 425 346**

**OPHTHALMIC RES., Band 19, Nr. 1, 1987, Seite 2: "Abstracts of the 27th Meeting of the Ass. for Eye Research, Oxford, GB, September 14-18, 1986"**

**KLIN. MBL. AUGENHEILK., Band 167, Nr. 1, Juli 1975, Seiten 88-93; F. Enke Verlag, Stuttgart, DE G. CREMER-BARTELS et al.: "Melatoninbiosynthesein der Säugetierretina in Abhängigkeit von der Adaptation an Belichtung."**

(73) Patentinhaber: **PHARM-ALLERGAN GMBH
Greschbachstrasse 1
W-7500 Karlsruhe 41(DE)**

(72) Erfinder: **Cremer, Gertrud, Prof. Dr.
Horstmarer Landweg 142
W-4400 Münster(DE)**

(74) Vertreter: **Brauns, Hans-Adolf, Dr. rer. nat. et al
Hoffmann, Eitle & Partner, Patentanwälte
Arabellastrasse 4
W-8000 München 81(DE)**

**EP 0 278 040 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

INVEST. OPHTHALMOL: VIS. SCI., Band 26, Nr. 5, Mai 1985, Seiten 768-770 G.N. RAO et al.: "Biosynthesis of neopterin, sepiapterin,and biopterin in rat and human ocular tissues."

CURR. EYE RES., Band 3, Nr. 2, Februar 1984, Seiten 273-277; IRL Press Ltd, Oxford, GB, K.B. MILLS et al.: "The effect of topical 0,5% triamterene suspension on the intraocular of open angle glaucoma patients. A single dose study."

DIALOG INFORMATION SERVICES, Biosis Previews, Band 69, Heft 5, September 1987, Ref.Nr. 13322598 G.G. Chusova et al.: "Changes in the uric-acid content in the blood of patients with retinitis pigmentosa and in rats with inherited retinal degeneration."

DIALOG INFORMATION SERVICES, Medline, Band 66, Heft 155, September 1987, Ref.Nr. 5484246 G.N. RAO et al.: "The enzymatic activities of GTP cyclohydrolase, sepiapterin reductase, dihydropteridene reductase and dihydrofolate reductase; and tetrahydrobiopterin content in mammalian ocular tissues and in human senile cataracts."

ANN. OTTALMOL. CLIN. OCUL., Band 97, Nr. 4, April 1971, Seiten 143-153, G. TOTA et al.: "L'elettroretinogramma dopo somministrazione di triamterene."

J. INVEST. DERMATOL., Band 80,Nr.4, Abstracts, Seite 320 S.LERMAN et al: "Further studies on allopurinol therapy and human cataradtogenesis."

THE MERCK MANUAL OF DIAGNOSIS AND THERAPY, 14. Edition, 1982, Seite 2005; Merck & Co., Inc., Rahway, N.J., US "Retinitis pigmentosa"

**Beschreibung**

Die Erfindung betrifft die Verwendung von Pteridinen und/oder Purinen als deren biochemischen Vorläufern oder von Xanthinoxidase-Hemmern zur Herstellung eines Arzneimettels für die Behandlung von genetisch bedingten degenerativen Netzhauterkrankungen, insbesondere Retinopathie pigmentosa (Retinopathia pigmentosa), nachfolgend mit RP bezeichnet.

In der nachfolgenden Beschreibung wird die Erfindung für die Behandlung von RP beschrieben, denn RP ist die häufigste degenerative Netzhauterkrankung. Man schätzt, daß weltweit 3 Millionen Menschen an einer der verschiedenen Formen von RP erkrankt sind.

Bei der RP handelt es sich um eine Erkrankung der Netzhaut, die im fortgeschrittenen Stadium zu Gesichtsfeldverlust und schließlich zur völligen Blindheit führen kann. Es ist ein degenerativer Prozeß, der meistens erblich ist. Im Verlauf der Krankheit findet eine Engstellung der Gefäße, eine Optikusatrophie, ein Zugrundegehen der nervösen Elemente der Netzhaut und eine Ablagerung von Pigment (knochenkörperchenartige Pigmentation), die von der Peripherie her bis zum Zentrum fortschreitet, statt.

Der Verlauf der RP von der ersten Symptomwahrnehmung bis zu starken Orientierungsschwierigkeiten dauert im Durchschnitt etwa 8 Jahre und beginnt häufig schon in der Pubertät. Im Gegensatz zum Normalsehenden mit einer Panoramasicht von ca. 180 bis 200° sieht der RP-Patient Gegenstände, die außerhalb der Blickrichtung liegen, nur ausschnittsweise oder gar nicht. Der Patient erlebt sehr bewußt, daß sich sein Gesichtsfeld ständig verkleinert. Er ist sich auch der Hoffnungslosigkeit seines Zustands sehr bewußt, denn weder eine Therapie zur Prophylaxe noch zur Behandlung oder sogar Heilung von RP ist bekannt. In der Regel sind die Patienten mit 40 bis 50 Jahren so stark beeinträchtigt, daß sie praktisch blind sind. Versuche, durch Verabreichung von 11-cis-Vitamin A das Fortschreiten von RP aufzuhalten, haben keine nachweisbaren Erfolge gezeigt. Man muß nach dem derzeitigen Wissensstand davon ausgehen, daß die Prognose von RP infaust ist.

Unter den vorgenannten Umständen liegt es auf der Hand, daß ein starkes Bedürfnis für ein Arzneimittel für die Behandlung von genetisch bedingten degenerativen Netzhauterkrankungen vom Typ der RP besteht.

In der US-A-4,425,346 werden pharmazeutische Zusammensetzungen beschrieben, die für topische Anwendung tetraazabicyclische Verbindungen enthalten. Sie dienen zur Behandlung von Glaukom und sind in der Lage, den intraokularen Druck zu vermindern. Bei einem Glaukom handelt es sich um den sogenannten "grünen Star". Dies ist ein Sammelbegriff für Krankheiten des Auges mit erhöhtem intraokularem Druck.

In KLIN. MBL. AUGENHEILK., Band 167, Nr. 1, Juli 1975, Seiten 88-93; F. Enke Verlag, Stuttgart, DE,
G. CREMER-BARTELS et al.: "Melatoninbiosynthese in der Säugetierretina in Abhängigkeit von der Adaptation an Belichtung."

wurde eine Erhöhung der Melatoninsynthese in vitro durch Triamteren beschrieben. Eine mögliche Verbindung einer gestörten Melatoninbiosynthese zum Stoffwechsel von zyklischen Nucleotiden wurde diskutiert, die wie derum im Zusammenhang mit retinalen Degenerationen stehen könnten. Dieser Zusammenhang wurde 1988 auf dem 8th International Congress of Eye Research von Farber et al. als nicht ursächlich dargelegt. Ihre Untersuchungen belegen, daß bei der rd Maus die vererbte retinale Degeneration nicht primär durch eine veränderte PDE (Phosphodiesterase) bedingt ist , die eine Schlüsselstellung im Metabolismus der zyklischen Nucleotide wahrnimmt. Weiterhin konnten immunzytochemische Untersuchungen von Wiechmann 1988 keine Hinweise auf eine Beteiligung des Melatoninstoffwechsels bei retinalen Degenerationen erbringen. Darüberhinaus stellt eine mögliche Beteiligung von Pterinen in der Pathogenese retionaler Dystrophien einen ganz anderen biochemischen Ansatzpunkt dar. Der Einfluß von Triamteren auf den Pterinstoffwechsel läuft vermutlich über eine Interaktion mit der Dihydropterinreductase, ein Stoffwechselweg, der in der o.a. Arbeit nicht Gegenstand von Untersuchungen war.

In OPHTHALMIC RES., Band 19, Nr 1., 1987, Seite 2:
"Abstracts of the 27th Meeting of the Ass. for Eye Research, Oxford, GB, September 14-18, 1986"
H. GERDING et al: "Pteridines of human blood in relation to degenerative retina diseases,"

wird festgestellt, daß Neopterin bei RP im Serum verringert gefunden wurde. Die Untersuchung wurde durchgeführt, um die Hypothese eines gestörten Immunsystems bei RP zu überprüfen. Aus dem Ergebnis ergab sich zunächst kein Hinweis auf einen therapeutischen Ansatz und dieser wurde in der Arbeit auch nicht als potentiell möglich diskutiert.

ANN. OTTALMOL. CLIN. OCUL., Band 97, Nr. 4, April 1971, Seiten 143-153,
G. TOTA et al.: "L'elettroretinogramma dopo somministrazione di triamterene."

berichtet über einen Anstieg der b-Welle im Elektroretinogramm kurzfristig nach Gabe von Triamteren. Dieses Phänomen wird vom Autor durch Elektrolytverschiebungen gedeutet, die unspezifisch in der Lage

sind, derartige Potentiale zu verändern. Es werden keinerlei Beziehungen zu dystrophischen Netzhauterkrankungen aufgezeigt; Aussagen über einen Effekt bei längerer Applikation, die dann einen möglichen spezifischen Einfluß dokumentieren könnten, fehlen.

Die vorliegende Erfindung basiert auf Untersuchungen, wonach bei Retinopathia pigmentosa-Patienten gewisse Pteridine gegenüber gesunden Normalpersonen signifikant erniedrigt sind. Deshalb liegt der vorliegenden Erfindung die neue Erkenntnis zugrunde, daß man bei Retinopathia pigmentosa-Patienten den Mangel an Pteridinen, der für die Pathogenese dieser Erkrankung von Bedeutung zu sein scheint, durch die Verabreichung von Pteridinen und/oder aber durch eine Beeinflussung der Pteridin-abbauenden Enzyme kompensieren kann.

Die Erfindung betrifft deshalb die Verwendung von Pteridinen und/oder Purinen als deren biochemischen Vorläufern oder eines Xanthinoxidase-Hemmers zur Herstellung eines Arzneimittels zur Behandlung von genetisch bedingten degenerativen Netzhauterkrankungen.

Allopurinol ist ein Urikostatikum, das als Xanthinoxidase-Hemmstoff der vermehrten Harnsäurebildung (Hyperurikämie) bei Gichtkranken entgegenwirkt. Allopurinol hat die Formel 1H-Pyrazolo[3,4-d]pyrimidin-4-ol

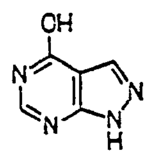

Es ist ein seit Jahren bewährtes und angewendetes Arzneimittel.

In den Pteridinen liegt ein mit einem Pyrimidinring kondensierter Pyrazinring vor. Pteridine kommen in der Natur vor und sind aus Schmetterlingsflügeln und auch aus Insektenaugen isoliert worden.

Die Pteridine sind aufgrund der in ihnen enthaltenen asymmetrischen Kohlenstoffatome Stereoisomere. In die Erfindung eingeschlossen sind die jeweiligen Diastereoisomeren und Razemate. Es ist bei pharmakologischen Wirkstoffen bekannt, daß häufig nur eine bestimmte diastereoisomere Form wirksam ist oder aber die größere Wirksamkeit gegenüber der anderen diastereomeren Form hat. Alle diese Formen sind in die vorliegende Erfindung eingeschlossen.

Als Pteridine, die für die vorliegende Erfindung geeignet sind, kommen beispielsweise in Frage: Monapterin, Sepiapterin, Xanthopterin. Nach dem derzeitigen Stand des Wissens wird Monapterin bevorzugt, jedoch ist davon auszugehen, daß aufgrund der biochemischen Umwandlung der einzelnen Pteridine in ein anderes Pteridin das Pteridin-Gerüst und die freie Verfügbarkeit von Pteridin im Körper wesentlich sind.

Triamteren ist ein mildes Diuretikum mit einem Pteridin-Gerüst. Deshalb kommt auch Triamteren für die Behandlung der RP in Frage. Ähnlich wie beim Allopurinol handelt es sich beim Triampteren um ein in der ärztlichen Praxis eingeführtes Arzneimittel - allerdings mit einer völlig anderen Indikation als bei der vorliegenden Erfindung - , so daß die Nebenwirkungen dieser Arzneimittel bereits bekannt sind und bei der Therapie berücksichtigt werden können.

Eingeschlossen in die vorliegende Erfindung sind auch die Säureadditionssalze der Pteridine. Die Pteridine sind in der Regel sehr schwer in Wasser löslich. Die Löslichkeit wird durch die Salzbildung verbessert. Obwohl anzunehmen ist, daß die salzfreie Form gewünschte Arzneimittelwirkung aufweist, können die Pteridine in Salzform, z.B. als Hydrochloridsalz, in den Arzneimitteln vorliegen, weil diese Salze im Stoffwechsel in die freie Form überführt werden. Die Schwerlöslichkeit in Wasser kann für die Ausbildung von Retardformen ausgenutzt werden. Dies ist insbesondere bei einer topischen Anwendung direkt im Auge von Bedeutung.

Purine sind Vorläufer für Pteridine. Deshalb wird auch im Rahmen der vorliegenden Erfindung die Applikation von solchen Vorläufern für die Behandlung von RP in Betracht gezogen und ist in die Erfindung eingeschlossen.

Die jeweiligen Dosierungsmengen können in einem weiten Bereich variiert werden. Die Schwerlöslichkeit der meisten Pteridine in Wasser und damit auch im Gewebe des menschlichen Körpers bei einer systemischen Wirkungsweise läßt eine breite Dosierung zu und wird in Abhängigkeit auch vom Gewicht und dem sonstigen Zustand des Patienten eingestellt. Ein- oder mehrmalige Verabreichung der oralen Applikationsformen pro Tag sind möglich.

Für den Fachmann ist es offensichtlich, daß in die jeweiligen Arzneimittelzubereitungen weitere in der Ophthalmologie bekannte Hilfsmittel und Wirkstoffe eingearbeitet werden können. stellvertretend für die

zahlreichen hier vorhandenen Möglichkeiten soll Benzalkoniumchlorid genannt werden.

Die Formulierung des Arzneimittels wird in der für die jeweilige Applikationsform üblichen und dem pharmazeuten und Galeniker vertrauten Weise durchgeführt. Für topische Anwendungen kommen zunächst die klassischen Formen von Augentropfen und Augensalben in Frage. Die Augentropfen bestehen z.B. aus einer Lösung oder Suspension des Wirkstoffes in Wasser für Injektionszwecke, Polyvinylalkohol als Trägersubstanz, Natriumchlorid, Natriumdisulfit, Kaliumhydrogenphosphat, Natriummonohydrogenphosphat, Natriumhydroxid sowie Benzalkoniumchlorid und Edetinsäure, Dinatriumsalz $2H_2O$ als Konservierungsstoffe.

Eine Augensalbe enthält vorzugsweise weißes Petrolatum (Vaseline®), flüssiges Paraffin, Amerchol®, Wasser für Injektionszwecke und Chlorbutanol zur Konservierung.

Da RP-Patienten auf eine dauernde Einnahme der hier genannten Wirkstoffe angewiesen sind, ist die schon vorerwähnte Retardform von besonderem Interesse, weil dadurch die Häufigkeit der Applikation in einer auch für die Patienten angenehmen Weise verringert werden kann. Für eine topische Retardform kommen Okularinserts, in denen sich die Wirkstoffe als Lösung, Suspension oder Feststoffe befinden, in Frage.

Tropfen und Salben müssen bei der Anwendung am Auge mehrmals täglich appliziert werden, da die freigesetzten Arzneistoffe bzw. die Arzneiform selbst durch die Lidbewegung und die Tränenflüssigkeit relativ rasch vom Applikationsort wieder entfernt werden. Anders verhalten sich die Einbettungen in Form von Okularinserten, die in den Bindehautsack für längere Zeit eingelegt werden. Ihre Lage wird durch Lidbewegung und Tränenflüssigkeit nicht beeinflußt. Durch die Wahl des Gerüstmaterials für die Einbettung kann die Freisetzung der Wirkstoffe beeinflußt und über längere Zeit konstant gehalten werden. Als Gerüstmaterial eignen sich physiologisch inerte Polymere, beispielsweise Gelatine, mit Formaldehyd gehärtete Gelatine, Polyphenylalkohol, Polyphenylpyrrolidon und auch Hydroxypropylmethylcellulose. Man kann diesen Polymeren Weichmacher zusetzen, um die Permeabilität und Elastizität der Okularinserte zu beeinflussen und den Erfordernissen anzupassen. Weichmacher sind beispielsweise Propylenglykole, Glycerin, Polyethylenglykol oder Triacetin.

Die Benetzbarkeit und Haftfähigkeit der Gerüstsubstanzen der Okularinserte können durch den Zusatz von amphiphilen Stoffen optimiert werden, beispielsweise durch Cholesterin oder andere physiologisch indifferente oberflächenaktive Substanzen.

Bei der Herstellung von oral applizierten Dosierungsformen können die üblichen Formen wie Kapseln, Tabletten, Pillen, Mehrschichttabletten, Dragees genannt werden. Für die Formulierung dieser oralen Applikationsformen werden die üblichen Hilfsstoffe verwendet, wie Träger- und Füllstoffe wie alpha-Lactose, Desintegrationsmittel wie Cellulose, Bindemittel wie Pektin und demineralisiertes Wasser. Die Kapseln kämen in Frage mit einer Hülle aus Stärke oder Gelatine scwie evtl. Geschmacksverbesserer.

Für eine parenterale Verabreichung kommen entsprechend zubereitete Lösungen für i.v. oder i.m. Verabreichung in Frage, wobei man in üblicher Weise flüssige Trägerstoffe wie Wasser, physiologische Kochsalzlösungen, Traubenzuckerlösungen etc. verwenden kann. Die parenteralen Zubereitungen können auch als Emulsionen oder Suspensionen zubereitet werden.

## Pharmakologische Untersuchungen

Es wurden Blutzellen von 25 RP-Patienten gewonnen; als Kontrollgruppe dienten 22 gesunde Normalpersonen und 26 Patienten mit Netzhautablösungen. Die Untersuchung ergab eine signifikante Erniedrigung des Monapterins in Lymphozyten und Erythrozyten.

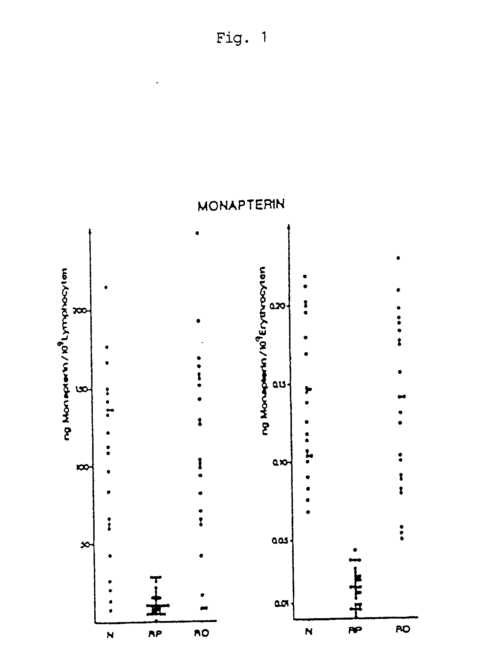

In der Fig. 1 wird der Gehalt von Monapterin in Erythrocyten und Lymphozyten von RP-Patienten, gesunden Normalpersonen und Patienten mit Netzhautablösungen (RD) gezeigt. Monapterin nahm signifikant ($p<0.001$) in den Lymphozyten und Erythrozyten von RP-Patienten im Vergleich zu den anderen Kontrollgruppen ab.

## Elektroretinographie bei Ratten

Jeweils acht Ratten (Longeveans) männlichen und weiblichen Geschlechts (16 Ratten in jeder Gruppe) erhielten Monapterin, bis zur Sättigung in Wasser gelöst, ad libidum während drei Wochen. Die andere Gruppe (16 Ratten) erhielt normales Leitungswasser.

Das Elekroretinogramm zeigte bei den Ratten, die Monapterin im Trinkwasser erhalten hatten, eine merkliche Veränderung.

## Beispiele

Beispiel 1

Okularinserte wurden in folgender Weise hergestellt (Mengenangaben in g):

| | |
|---|---|
| Polyvinylalkohol | 0,6 |
| Polyvinylpyrrolidon | 0,2 |
| Cholesterin | 0,05 |
| Propylenglykol | 0,2 |
| L-Monapterin | 0,1 |
| Ethylalkohol (96 %ig) | 2,0 |
| Wasser für Injektionszwecke | 10,0 |

Unter sterilen Bedingungen wird Polyvinylalkohol in dem auf 70 °C erwärmten Wasser gelöst. Cholesterin, Polyvinylpyrrolidon, der Wirkstoff und Propylenglykol werden in Ethylalkohol gelöst und die alkoholische Lösung zu der wäßrigen Lösung gegeben. Der Ansatz wird auf eine mit PTFE-beschichtete Platte gegossen und das Lösungsmittel abgedampft. Aus dem gebildeten Film werden ellipsoide Okularinserte von etwa 0,8 $cm^2$ gestanzt.

Beispiel 2

Für etwa 10 ml Suspension werden jeweils in g verwendet:

| | |
|---|---|
| Methylcellulose | 0,05 |
| L-Monapterin | 0,1 |
| Chloramphenicol | 0,05 |
| Wasser für Injektionszwecke | 10,0 |

**Patentansprüche**

1. Verwendung von Pteridinen und/oder Purinen als deren biochemischen Vorläufern oder eines Xanthinoxidase-Hemmers zur Herstellung eines Arzneimittels zur Behandlung von genetisch bedingten degenerativen Netzhauterkrankungen.

2. Verwendung gemäß Anspruch 1 zur Behandlung von Retinopathie pigmentosa.

3. Verwendung gemäß Anspruch 1 oder 2, dadurch **gekennzeichnet**, daß man als XanthinoxidaseHammer Allopurinol verwendet.

4. Verwendung gemäß Anspruch 1 oder 2, dadurch **gekennzeichnet**, daß man als Pteridin Monapterin, Sepiapterin oder Triamteren verwendet.

5. Verwendung gemäß einem vorhergehenden Ansprüche, dadurch **gekennzeichnet**, daß das Arzneimittel in einer oralen Applikationsform vorliegt.

6. Verwendung gemäß einem der vorhergehenden Ansprüche, dadurch **gekennzeichnet**, daß das Arzneimittel in Form von topisch applizierbaren Augentropfen (Lösung, Suspension, Emulsion, Salbe) vorliegt.

7. Verwendung gemäß Anspruch 6, dadurch **gekennzeichnet**, daß der Wirkstoff in Form eines Okularinserts vorliegt.

8. Verwendung gemäß Anspruch 7, dadurch **gekennzeichnet**, daß der Wirkstoff in dem Okularinsert in Retardform vorliegt.

9. Verwendung gemäß Anspruch 8, dadurch **gekennzeichnet**, daß die Retardform in Form eines - schwer wasserlöslichen Salzes vorliegt.

**Claims**

1. The use of pteridines and/or purines as the biochemical precursors thereof or of a xanthinoxidase inhibitor for the production of a pharmaceutical preparation for the treatment of genetically caused, degenerative retina diseases.

2. The use as claimed in Claim 1 for the treatment of retinopathia pigmentosa.

3. The use as claimed in Claim 1 or 2, characterised in that allopurinol is used as xanthinoxidase inhibitor.

4. The use as claimed in Claim 1 or 2, characterised in that monapterin, sepiapterin or triamterene is used as pteridine.

5. The use as claimed in one of the preceding claims, characterised in that the pharmaceutical preparation has an oral form of administration.

6. The use as claimed in one of the preceding claims, characterised in that the pharmaceutical preparation has the form of topically administrable eye drops (solution, suspension, emulsion, salve).

7. The use as claimed in Claim 6, characterised in that the active ingredient has the form of an ocular insert.

8. The use as claimed in Claim 8, characterised in that the active ingredient is present in slow-release form in the ocular insert.

9. The use as claimed in Claim 8, characterised in that the slow release form is a salt having low solubility in water.

**Revendications**

1. Utilisation de ptéridines et/ou purines, sous la forme de leurs précurseurs biochimiques, ou d'un inhibiteur de la xanthine oxidase pour la préparation d'un médicament pour le traitement des maladies liées génétiquement à la dégénérescence de la rétine.

2. Utilisation selon la revendication 1, pour le traitement de la rétinopathie pigmentosa.

3. Utilisation selon la revendication 1 ou 2, caractérisée par le fait que l'on utilise l'allopurinol comme inhibiteur de la xanthine oxidase.

4. Utilisation selon la revendication 1 ou 2, caractérisée par le fait que l'on utilise comme ptéridine le monaptérine, la sepiaptérine ou le triamtérène.

5. Utilisation selon l'une des revendications précédentes, caractérisée par le fait que le médicament se présente sous une forme à application orale.

6. Utilisation selon l'une des revendications précédentes, caractérisée par le fait que le médicament se présente sous une forme de gouttes pour les yeux, à application topique (solution, suspension, émulsion, onguent).

7. Utilisation selon la revendication 6, caractérisée par le fait que la substance active se présente sous forme d'insert oculaire.

8. Utilisation selon la revendication 7, caractérisée par le fait que la substance active se présente dans l'insert oculaire sous une forme retardée.

9. Utilisation selon la revendication 8, caractérisée par le fait que la forme retardée se présente comme un sel difficilement soluble dans l'eau.

Fig. 1